(19) Europäisches Patentamt
European Patent Office
Office européen des brevets

(11) Publication number: 0 112 378 B1

(12) # EUROPEAN PATENT SPECIFICATION

(45) Date of publication of patent specification: 11.01.89

(51) Int. Cl.$^4$: A 61 B 1/00

(21) Application number: 83902333.0

(22) Date of filing: 02.06.83

(86) International application number: PCT/US83/00851

(87) International publication number: WO 84/00101 19.01.84 Gazette 84/02

(54) **ELECTRO-OPTICAL DEVICE FOR MONITORING INSTANTANEOUS SINGLET OXYGEN CONCENTRATION PRODUCED DURING THE TREATMENT OF CANCER BY MEANS OF PHOTOCHEMOTHERAPY.**

(30) Priority: 28.06.82 US 393188

(43) Date of publication of application: 04.07.84 Bulletin 84/27

(45) Publication of the grant of the patent: 11.01.89 Bulletin 89/02

(84) Designated Contracting States: DE FR GB SE

(56) References cited:
EP-A-0 003 015
DE-A-2 813 317
US-A-3 123 066
US-A-3 136 310
US-A-3 958 560
US-A-4 336 809

(73) Proprietor: THE JOHNS HOPKINS UNIVERSITY
Applied Physics Laboratory Johns Hopkins Road
Laurel, MD 20707 (US)

(72) Inventor: PARKER, John G.
1705 Howe Drive
Olney, MD 20832 (US)
Inventor: STANBRO, William D.
10916 Batter Sea Lane
Columbia, MD 21044 (US)

(74) Representative: UEXKÜLL & STOLBERG
Patentanwälte
Beselerstrasse 4
D-2000 Hamburg 52 (DE)

(56) References cited:
Rev. Sci. Instrum. Vol. 51, No. 10., October 1980, Kinsen et al, Endoscopic System for simultaneous electronic detection of fluorescence, pp. 1403-1406.
Cancer Research, Vol. 38, August 1978, Dougherty et al, Photoradiation Therepy for the Treatment of Malignant Tumors, pp. 2628-2635
Chest, Vol. 76:1, July 1979, Doiron et al, Fluorescence Bronchoscopy for Detection of Lung Cancer, pp. 27-32.

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 112 378 B1

**Description**

Background and/or environment of the invention

1. Field of the invention

This invention relates to an apparatus and method for monitoring the instantaneous concentration of singlet oxygen produced during photochemotherapy and thereby provides a tool to assess the effectiveness of the photochemotherapy treatment and prescribe a correct dosage of therapeutic photoradiation.

2. Background and technical summary

When certain hematoporphyrin preparations are injected intravenously into the human body, they are selectively retained by cancerous tissue. Thus, two or three days after injection, significantly higher levels of hematoporphyrin accumulate in malignant tissue. The selective retention of hematoporphyrin by cancerous tissue was first used clinically as a diagnostic tool. In the presence of ultraviolet or shortwave length visible light, such tissue will exhibit a bright red light fluorescence while normal tissue appears light pink. A discussion of clinical investigations using this diagnostic technique can be found in an article entitled "Hematoporphyrin Diacetate: A Probe to Distinguish Malignant from Normal Tissue by Selective Fluorescence" by R. W. Henderson, G. S. Christie, P. S. Clezy and J. Lineham, *Brit. J. Exp. Pathol.,* Vol. 61, pages 325—350 (1980). Another reference by D. R. Doiron and A. E. Profio entitled "Laser Fluorescence Bronchoscopy for Early Lung Cancer Localization" published in *Lasers in Photomedicine and Photobiology* (1980) teaches the use of a laser fluorescence bronchoscope to detect and localize small lung tumors by observing this red fluorescence.

An additional clinical application has recently been found in the treatment and destruction of malignant tissue, i.e., photochemotherapy or photoradiation therapy. The process by which biological damage occurs, as the result of optical excitation of a hematoporphyrin dye in the presence of oxygen, is generally referred to as "photodynamic action". As indicated above, photochemotherapy involves the injection of a hematoporphyrin dye intravenously into the patient. After the passage of several days, usually three, the dye is retained in significant amounts by cancerous tissue, however being eliminated by healthy tissue. The tumor is then exposed to a therapeutic light and this light energy causes the photodynamic dye to be excited to a high energy state. Through a direct intramolecular process, the dye transfers energy to oxygen molecules present in the tissue and raises them from the ground triplet to the first excited electronic singlet state, $^1O_2$. The singlet oxygen, $^1O_2$, attacks and functionally destroys the cell membranes ultimately inducing necrosis and destroying the cancerous tissue.

In an article by Thomas J. Dougherty et al entitled "Photoradiation Therapy for the Treatment of Malignant Tumors" published in *Cancer Research*, Vol. 38, pages 2628—2635 (1978), problems associated with prescribing the correct therapeutic light dosages are discussed. If the dosage is too weak, the tumor response will be partial or incomplete. If the irradiation time is too long or intensity too high, normal skin or tissue will experience necrosis. The aforementioned article points out the difficulty of determining the correct therapeutic dosage of light. This problem has been proven to be quite significant and currently is one of the major hurdles that must be overcome before photochemotherapy can be used to treat deep-seated large tumors.

Summary of the invention

The current inventors recognize that adequate treatment is questionable unless one knows directly the rate at which singlet oxygen is generated in the malignant tissue. The inventors recognize that the generation of singlet oxygen involves several key factors: (1) the therapeutic light source must be of the proper intensity and wavelength sufficient to elevate the photodynamic dye to an excited singlet state; (2) there must be a sufficient concentration of oxygen localized within the tumor both initially and during the period of irradiation to be excited by energy transfer from the dye; (3) the local dye concentration may vary considerably, depending on the hydrophobic or hydrophilic nature of the immediate environment; and (4) the rate of quenching of $^1O_2$ may vary substantially with the existing medium, being generally more rapid in an aqueous than in a lipid environment. Therefore, merely measuring the incident light intensity and time of exposure will not supply adequate information regarding the generation of singlet oxygen within the tumor tissue. The inventors, therefore, recognize the need to determine directly the amount of singlet oxygen which is being generated during the light excited hematoporphyrin process.

The inventors recognize that direct monitoring is possible because in the interaction of single oxygen molecules, with the surrounding medium, the singlet oxygen molecules undergo a radiative transition to its ground triplet state emitting light at a wavelength of 1.27 micros, e.g., J. G. Parker and W. D. Stanbro, "Optical Determination of the Collisional Lifetime of Singlet Molecular Oxygen [$O_2{}^1\Delta_g$] in Acetone and Deuterated Acetone", *J. Am. Chem. Soc.,* Vol. 104, pp. 2067—2069 (1982). The singlet oxygen emission was first described in an article by A. A. Krasnovsky, Jr. entitled "Photosensitized Luminescence of Singlet Oxygen in Solution" reprinted in *Biophysics* (GB), Vol. 21, page 770 (1976). Krasnovsky is generally recognized as the first to identify and optically detect the singlet oxygen emission at 1.27 microns in solution. Krasnovsky used a cryogenically cooled photomultiplier as the means for detecting this emission in a $CCl_4$ solvent. Additional work has been done for various other solvents, e.g., A. H. Khan and M. Kasha, "Direct Spectroscopic Observation of Singlet Oxygen

Emission at 1268 nm Excited by Sensitizing Dyes of Biological Interest in Liquid Solution", *Proc. Natl. Acad. Sci.,* Vol. 76(12), page 6047 (1979); and K. I. Salokhiddinov, B. N. Dzhagarov, I. M. Byteva, and G. P. Gurinovich, "Photosensitized Luminescence of Singlet Oxygen in Solutions at 159 nm", *Chem. Phys. Lett.,* Vol. 76(1), page 85 (1980). To detect the 1.27 microns emission Khan et al used a near-infrared spectrophotometer that employed a thermoelectrically cooled lead sulfide detector and Salokhiddinov et al used a liquid nitrogen cooled germanium photodiode.

The present inventors have developed a method and apparatus for measuring singlet oxygen generated during photochemotherapy by using an optical sensor. This method and apparatus has applications for various types of tumors: for skin tumors the optical sensor can be placed near the skin surface to receive the 1.27 micron radiation emitted from the tumor; for deep seated intracorporeal tumors a fiberoptical probe is inserted into the tumor mass and conveys the radiation to the optical sensor; and, for eye tumors a fiberoptic/contact lens arrangement can be used to collect the 1.27 micron radiation and convey it to the optical sensor. The optical sensor generally consists of a photodiode which is preceded by an infrared band-pass filter to provide the necessary rejection. The photodiode most suited for this application is an epitaxial unit composed of indium, gallium and arsenic (InGaAs). These detectors have a much better time response characteristic than germanium and since they are relatively insensitive to excitation below 9000Å they can provide significant discrimination against the visible exciting light. The superiority of the InGaAs device both in regard to temporal discrimination required in separating the short duration exciting light from the more slowly varying $^1O_2$ emission and also in spectral discrimination establishes it as the preferable detector for this application.

To provide the necessary signal-to-noise enhancement, the photodiode output is fed directly to either a lock-in amplifier or first amplified and then fed to a box-car integrator. The particular choice depends on temporal characteristics of the incident therapeutic light. After processing, the signal is fed to an indicator to provide an instantaneous reading of the average singlet oxygen level within the tumor mass. By monitoring the singlet oxygen level, the physician or operator can adjust the therapeutic irradiation conditions (i.e., intensity, wavelength, angle of incidence, time of exposure, etc.) to provide a singlet oxygen level as necessary to satisfy predetermined clinical conditions. The invented method and apparatus thus provides a means for remotely monitoring the total singlet oxygen production, a production rate which may vary substantially with local physiological conditions.

Brief description of the drawings

Figure 1 is a graph illustrating the overall system kinetics showing therapeutic light stimulation of photosensitizing dye and the resulting generation of singlet oxygen.

Figure 2 illustrates, in block diagrammatic form, the invented apparatus used to monitor singlet oxygen generated in skin tumors when pulsed therapeutic light is used.

Figure 3 illustrates, in block diagrammatic form, the invented apparatus used to monitor singlet oxygen generation in skin tumors when substantially continuous therapeutic light is used.

Figure 4 illustrates, in block diagrammatic form, the invented apparatus used to monitor singlet oxygen generation in deep seated tumors when pulsed therapeutic light is used.

Figure 5 illustrates, in block diagrammatic form, the invented apparatus used to monitor singlet oxygen generation in deep seated tumors when substantially continuous therapeutic light is used.

Figure 6 illustrates a contact lens/fiberoptic link arrangement for monitoring singlet oxygen generation in eye tumors.

Description of the preferred embodiments

The mechanism by which laser-induced photodynamic action destroys malignant tissue appears to involve a two-step process. Figure 1 illustrates schematically the overall system kinetics. First the photodynamic dye is excited from the ground state, $S_0$, to the first excited electronic state, $S_1$, by means of a therapeutic light source, such as pulsed laser radiation at 5320 angstroms (green light). The dye singlet state $S_1$ is then transformed via an intramolecular coupling to the lowest-lying dye triplet state, $T_0$, as indicated in the figure. Subsequent deactivation of this triplet state to the ground singlet state, $S_0$, in the absence of oxygen, is forbidden by the spin selection rules, resulting in lifetimes of this particular species under anaerobic (oxygen-excluded) conditions as long as one millisecond. However, in the presence of oxygen localized in the tissue, the dye triplet, $T_0$, will deactivate rapidly (0.3 to 3.0 $\mu_{sec}$) to the dye singlet ground state, $S_0$, in a spin-conserving intramolecular process in which a sizeable fraction of the energy in the dye triplet is transferred to the triplet electronic state of oxygen, $^3O_2$, raising the oxygen to the first excited electronic singlet state, $^1O_2$, approximately 1 electron-volt above the ground state. This spin-conserving energy transfer is possible only because the ground electronic state of molecular oxygen happens to be a triplet which is not true for most stable molecules. This energy transfer, which is shown un Figure 1, can be represented as follows:

$$(1) \qquad T_0+{}^3O_2 \rightarrow S_0+{}^1O_2$$

When singlet oxygen, $^1O_2$, interacts with molecules contained in the surrounding medium M, a relatively weak collision-induced emission occurs:

(2) $^1O_2+M \rightarrow {}^3O_2+M+h\sim_E$.

The quantity ~E is the emission frequency, and corresponds to a wavelength of 1.27 microns. By optically detecting and monitoring this emission it is possible to determine the instantaneous rate at which the active cytotoxic agent, singlet oxygen, is being generated and is attacking the malignant tissue.

The specific site of singlet oxygen attack has been identified as the cell membrane. Significant differences in hematoporphyrin accumulation and membrane damage have been found to occur, depending on the hydrophobic or hydrophilic nature of the specific membrane site and compensating solubility of the photosensitizer. It will be noted that several photosensitizing dyes, such as rose bengal, eosin-y, fluorescein, methylene blue, other than hematoporphyrin, may be used to produce the above-described photodynamic action. Hematoporphyrin, however, has the advantage of being relatively non-toxic and has also evidenced itself to be resistant to oxidation, thus quite stable.

Current photochemotherapy treatment involves intravenous injection of the patient with hematoporphyrin. After two to three days the hematoporphyrin has been selectively retained by the tumor tissue. Photoradiation is then used to excite the hematoporphyrin to its singlet state and, by the process illustrated in Equation 1, produces singlet oxygen within the malignant tissue. The therapeutic light may penetrate directly through the patient's skin into the tumor area if the tumor is located relatively close to the surface. For deep-seated or large tumors, a system employing fiberoptics may be used which allows delivery of the light to any desired depth within the tumor. The therapeutic light can also be delivered by various types of endoscopes to the cervix, bronchus, bladder, etc.

However, as mentioned previously, the problem with the prior art is that the correct intensity and duration of the therapeutic light dosage can't be determined. The physician must guess at the correct therapeutic light dosage. If the dosage is too high, normal tissue will experience necrosis; and, if the dosage is insufficient, the tumor will not be destroyed. Since the physician has no way of knowing the rate at which oxygen is being delivered to the tissue or the magnitude of the local dye concentration, it is impossible to predict the effect of a given therapeutic light dosage. If the light dosage is too intense, local oxygen may be depleted more rapidly than it is provided to the tissue and no additional singlet oxygen will be generated for any increase in intensity or duration of the light dosage. The only adequate way to prescribe the therapeutic light dosage therefore is by direct monitoring of the level of singlet oxygen produced. As has been mentioned, remote optical detection of the singlet oxygen level is possible because of the existence of a collisionally induced infrared emission at a wavelength of 1.27 microns as indicated in Equation 2.

Figures 2 and 3 illustrate, in block diagrammatic form, the invented apparatus used to monitor singlet oxygen concentration during the treatment of skin tumors or tumors near the skin surface. The apparatus generally contains: an optical detection means 10 which collects light emitting from the tumor and detects the intensity of such light at the 1.27 micron wavelength band; an amplification means 12 which amplifies and processes electrical signals generated by the optical detection means 10; and, an output indicator 14 which indicates the instantaneous singlet oxygen concentration occurring during photochemotherapy treatment.

In operation, therapeutic light (pulsed 16 or substantially continuous 18) is used to irradiate the tumor site 20. The tumor has been previously treated and has absorbed a photosensitizing dye, such as hematoporphyrin. The therapeutic photoradiation produces singlet oxygen, the active cytotoxic agent, which emits radiation in a relatively narrow spectral band centered at a wavelength of 1.27 microns. The optical detection means 10 collects and detects the 1.27 micron emission, and produces an electrical signal which is amplified by the amplification means 12. The output indicator 14 connects to the amplification means 12 and displays the level of instantaneous singlet oxygen concentration. The instantaneous concentration level could be used to further determine the maximum level of singlet oxygen produced or the quenching rate. The instantaneous concentration level could be used to adjust the dosage of the therapeutic radiation, i.e., indicate whether the intensity, duration, frequency or modulation of the therapeutic radiation should be modified for more effective photochemotherapy of the particular tumor or a particular part of the tumor.

Figure 2 illustrates the invented apparatus when a pulsed or chopped therapeutic light 16 is used. The apparatus consists of: a lens arrangement 22 for collecting and concentrating light generated in the tumor; a band pass filter 24 which filters unwanted light and passes light in the 1.27 micron wavelength band; a photodetector 26 which detects light passed by the filter 24; a preamplifier 28 and a boxcar integrator (or transient recorder combined with a signal averager) 30 to amplify and process the electrical signal produced by the photodetector 26; and, an output indicator 14 which displays the instantaneous singlet oxygen concentration and/or quenching rate.

The photodetector 26 must be able to detect weak emissions at a wavelength of 1.27 microns. The spectral response of the diode photodetector combined with the bandpass filter 24 must be such that the therapeutic light, which appears in the visible part of the spectrum, and the red fluorescent light, which appears when the dye undergoes a radiative transition from its excited singlet to the ground singlet state, are both filtered out. The photodetector 26 must also be fast enough to be responsive to time varying intensity of the light emission. The inventors prefer an indium-gallium-arsenide (InGaAs) epitaxial device because it is

selectively responsive to infrared emission in the desired wavelength range with minimal filtration requirements and because of its rapid time response. This type of device is used extensively in commercial fiberoptic transmission systems.

Figure 3 illustrates the invented apparatus when a substantially continuous intensity of therapeutic light 18 is used. The apparatus consists of: a lens arrangement 22 for collecting and concentrating light generated in the tumor; a mechanical or electro-optical chopper 32 which is inserted for signal to noise enhancement; a band pass filter 24 which filters unwanted light frequencies and passes light in the 1.27 micron wavelength band; a photodetector 26 which detects light passed by the filter 24; a lock-in amplifier 34 which amplifies and processes the electrical signal generated by the photodetector 26; and, an output indicator 14 which displays the instantaneous level of singlet oxygen concentration. As discussed above, the photodetector 26, may be an InGaAs diode.

Figures 4 and 5 illustrates, in a block diagrammatic form, the invented apparatus used to monitor singlet oxygen concentration during the treatment of deep seated intracorporeal tumors. The apparatus generally contains: an optical detection means 10 which collects and detects light in the 1.27 microns wavelength band emitting from deep seated tumors; an amplification means 12 to amplify and process the signal produced by the optical detecting means 10; and, an indicator means 14, which indicates the instantaneous singlet oxygen concentration.

If the therapeutic light 16 is pulsed, or if a substantially continuous therapeutic light is optically chopped, the resulting generation and decay of the singlet oxygen molecules will also result in a pulsed emission. Figure 4 illustrates the invented apparatus when a pulsed therapeutic light 16 is used. The apparatus consists of: a fiberoptic waveguide 36 which is inserted into the tumor mass 38 through a hollow hypodermic needle 40 to collect light emitted within the tumor; a bandpass filter 24 which filters unwanted light frequencies and passes light in the 1.27 micron wavelength band; a photodiode 26 which detects light passed by the filter 24 and may have characteristics which also filters out unwanted light frequencies; a preamplifier 28 which amplifies electrical signals generated by the photodetector 26; a boxcar integrator (or transient recorder combined with a signal averager) 30, to further process the signal; and, an outut indicator 14 which displays the instantaneous level of slight oxygen concentration and or quenching rate. As discussed above the photodetector 26 may be a InGaAs diode.

Figure 5 illustrates the embodiment when a substantially constant intensity therapeutic light source 28 is used. The apparatus consists of: a fiberoptic waveguide 36 which is inserted into the tumor mass 38 through a hollow hypodermic needle 40 to collect light emitted within the tumor; a mechanical or electro-optical chopper 42 is inserted along the fiberoptic waveguide 36; a bandpass filter 24 which filters unwanted light frequencies, and passes light in the 1.27 microns band; a photodetector 26 which detects light passed by the filter 24 and has characteristics which further filters out unwanted light frequencies; a lock-in amplifier 34 which amplifies and processes the electrical signal generated by the photodetector 26; and, an output indicator 14 which displays the instantaneous level of signal oxygen concentration. The photodetector 26 may be a InGaAs diode and the chopper 42 may be an optoacoustic modulator.

The fiberoptic waveguide 36 can be inserted into the cancerous tissue via a hollow hypodermic needle 40. In another embodiment, it might be preferable to have the therapeutic light source also enter the cancerous tumor through a fiberoptic link. In this event the fiberoptic carrying the therapeutic light and the fiberoptic probe link could be coupled and inserted simultaneously into the cancerous tumor. A hypodermic needle or an endoscope would be appropriate for this application.

In operation, the hypodermic needle 40 would permit penetration of the fiberoptic waveguide 36 into the area of the tumor mass to monitor the singlet oxygen generation. Since light at a wavelength of 1.27 microns penetrates more readily through living tissue than does visible light, the exact placement of the fiberoptic probe in the tumor mass is not critical. The intensity, frequency, and modulation of the therapeutic light (pulsed 16 or substantially continuous 18) can be adjusted in response to the singlet oxygen level. In this manner the invented apparatus provides a practical way to measure the effect of the incident therapeutic light dosage and makes quantitative photochemotherapy possible as a means of clinical cancer treatment.

In the treatment of eye tumors, the fiberoptic link 36 shown in Figures 4 and 5 can be optically coupled to a contact lens rather than inserted into the tumor area. The contact lens arrangement, shown in Figure 6, generally contains a contact lens 44 which is placed over the cornea and optically coupled to a fiberoptic link 36. The contact lens 44 collects the 1.27 micron emission generated by singlet oxygen during photoradiation of eye tumors. A single fiberoptic link coupled to the contact lens can transmit therapeutic light into the eye and collect the 1.27 micron emission coming from the eye; or a bundle of fiberoptic links can be optimally coupled to the contact lens and one of the links can collect light emitted from the eye tumor.

Obviously many modifications and variations of the present invention are possible in light of the above teachings. It is therefore to be understood that within the scope of the appended claims the invention may be practiced otherwise than as specifically described.

## Claims

1. An apparatus for monitoring the instantaneous singlet oxygen concentration produced during the treatment of cancer by means of photochemotherapy, said photochemotherapy treatment involving therapeutic radiation of cancerous tissue which has absorbed a photosensitizing dye, characterized by

an optical detection means (10) for detecting the intensity of light emissions in the 1.27 micron wavelength band caused by collisionally induced radiative deactivation of singlet oxygen, said light emissions produced in said cancerous tissue during photochemotherapy, said optical detection means producing an electrical output signal;

amplification means (12, 34), connected to said optical detection means, for amplifying and processing said electrical output signal; and,

an output indication means (14), connected to said amplification means, for indicating the instantaneous concentration of singlet oxygen.

2. The apparatus of claim 1, wherein said optical detection means comprises:

a filter means (24) for selecting said light emissions in said 1.27 micron wavelength band by filtering unwanted light radiation; and,

a photodetector means (26), optically coupled to said filter means, for detection of the intensity of said light emission in said 1.27 micron wavelength band.

3. The apparatus of claim 2, wherein said photodetector means (26) is an InGaAs photodiode.

4. The apparatus of claim 2, wherein said optical detection means (10) further comprises a means (22, 36, 44) for collecting said light emissions produced in said tissue during photochemotherapy, the output from said collection means being optically coupled to said filter means (24).

5. The apparatus of claim 4, wherein said collection means is a fiberoptic link (36).

6. The apparatus of claim 4, wherein said collection means is a contact lens (44) optically coupled to said filtering means by a fiberoptic link (36).

7. The apparatus of claim 4, wherein said collection means is a lens arrangement.

8. The apparatus of claim 4, wherein said therapeutic radiation (16) is pulsed and wherein said amplification means comprises:

a preamplifier (28) connected to said photodetector means (26), for amplifying said electrical signals generated by said photodetector means; and

a boxcar integrator (30), connected to said preamplifier (28) for processing the output from said preamplifier.

9. The apparatus of claim 4, wherein said therapeutic radiation (18) is substantially continuous, said optical detection means further comprises a chopper (32, 46) optically coupled to the output of said collection means and optically coupled to the output of said filter means and wherein said amplification means comprises a lock-in amplifier (34).

10. The apparatus of claim 9, wherein said chopper is an optoacoustic modulator.

11. The apparatus of claim 2 wherein said therapeutic radiation (18) is substantially continuous, and said optical detection means includes a chopper (46) optically coupled to the input of said filter means (24).

12. The apparatus of claim 5, wherein said fiberoptic link (36) is inserted into said cancerous tissue through a hollow hypodermic needle (40).

## Patentansprüche

1. Vorrichtung zum Anzeigen der momentanen Singulet-Sauerstoff-Konzentration, die während der Krebsbehandlung durch Fotochemotherapie erzeugt wird, wobei die Fotochemotherapie die therapeutische Bestrahlung des krebsartigen Gewebes umfaßt, das einen fotoempfindlich machenden Farbstoff absorbiert hat, gekennzeichnet durch:

eine optische Anzeigeeinrichtung (10) zur Feststellung der Intensität der Lichtemissionen im 1,27 µ Wellenlängenband aufgrund von durch Stoß induzierter strahlender Deaktivierung von Singulet-Sauerstoff, wobei die Lichtemissionen in dem krebsartigen Gewebe während der Fotochemotherapie erzeugt werden und die optische Erkennungseinrichtung ein elektrisches Ausgangssignal erzeugt;

Verstärkungseinrichtungen (12, 34), die an die optische Erkennungseinrichtung angeschlossen sind, um das elektrische Ausgangssignal zu verstärken und zu verarbeiten; und

eine Ausgabeanzeigeeinrichtung (14), die an die Verstärkungseinrichtungen angeschlossen sind, um die momentane Konzentration des Sigulet-Sauerstoffs anzuzeigen.

2. Vorrichtung nach Anspruch 1, wobei die optische Erkennungseinrichtung aufweist:

eine Filtereinrichtung (24) zum Selektieren der Lichtemissionen in dem 1,27 µ Wellenlängenband durch Filterung von unerwünschter Lichtstrahlung; und

eine Fotodetektoreinrichtung (26), die mit der Filtereinrichtung optisch gekoppelt ist, um die Intensität der Lichtemission in dem 1,27 µ Wellenlängenband festzustellen.

3. Vorrichtung nach Anspruch 2, worin die Fotodetektoreinrichtung (26) eine InGaAs-Fotodiode ist.

4. Vorrichtung nach Anspruch 2, wobei die optische Erkennungseinrichtung (10) ferner eine Einrichtung (22, 36, 44) aufweist, um die in dem Gewebe während der Fotochemotherapie erzeugten Lichtemissionen zu sammeln, wobei die Ausgabe aus der Sammeleinrichtung optisch auf die Filtereinrichtung (24) gekoppelt wird.

5. Vorrichtung nach Anspruch 4, wobei die Sammeleinrichtung eine Faseroptikverbindung (36) ist.

6. Vorrichtung nach Anspruch 4, wobei die Sammeleinrichtung eine Kontaktlinse (44) ist, die

an die Filtereinrichtung durch eine Faseroptikverbindung (36) optisch angekoppelt ist.

7. Vorrichtung nach Anspruch 4, wobei die Sammeleinrichtung eine Linsenanordnung ist.

8. Vorrichtung nach Anspruch 4, wobei die therapeutische Strahlung (16) gepulst ist und wobei die Verstärkungseinrichtung aufweist:

einen Vorverstärker (28), der an die Fotodetektoreinrichtung (26) angeschlossen ist, um die von der Fotodetektoreinrichtung erzeugten elektrischen Signale zu verstärken; und

einen Impulsspitzenintegrator (30), der an den Vorverstärker (28) angeschlossen ist, um die Ausgabe aus dem Vorverstärker zu verarbeiten.

9. Vorrichtung nach Anspruch 4, worin die therapeutische Strahlung (18) im wesentlichen kontinuerlich ist, und wobei die optische Erkennungseinrichtung ferner einen Chopper (32, 46) aufweist, der an den Ausgang der Sammeleinrichtung optisch angekoppelt und auch an den Ausgang der Filtereinrichtung optisch angekoppelt ist, und wobei die Verstärkungseinrichtung einen Einfangverstärker (34) aufweist.

10. Vorrichtung nach Anspruch 9, wobei der Chopper ein optoakustischer Modulator ist.

11. Vorrichtung nach Anspruch 2, wobei die therapeutische Strahlung (18) im wesentlichen kontinuierlich ist, und wobei die optische Erkennungseinrichtung einen Chopper (46) aufweist, der an den Eingang der Filtereinrichtung (24) optisch angekoppelt ist.

12. Vorrichtung nach Anspruch 5, wobei die Faseroptikverbindung (36) durch eine hohle Spritzennadel (40) in das krebsartige Gewebe eingesetzt wird.

**Revendications**

1. Appareil pour contrôler la concentration instantanée d'oxygène singulet produit pendant le traitement d'un cancer par photochimiothérapie, ledit traitement par photochimiothérapie impliquant l'irradiation thérapeutique de tissus cancéreux ayant absorbé un colorant photosensibilisateur, caractérisé par

un moyen de détection optique (10) pour détecter l'intensité d'émissions lumineuses dans la bande de longueur d'onde de 1,27 micromètre provoquées par la désactivation radiative d'oxygène singulet induite par des collisions, lesdites émissions lumineuses étant produites dans lesdits tissues cancéreux pendant la photochimiothérapie, ledit moyen de détection optique produisant un signal électrique de sortie;

un moyen d'amplification (12, 34), relié audit moyen de détection optique, pour amplifier et transformer ledit signal électrique de sortie; et

un moyen d'indication (14) de sortie, relié audit moyen d'amplification, pour indiquer la concentration instantanée d'oxygène singulet.

2. Appareil selon la revendication 1, dans lequel ledit moyen de détection optique comprend:

un moyen formant filtre (24) pour sélectionner lesdites émissions lumineuses dans ladite bande de longueur d'onde de 1,27 micromètre par filtrage des rayonnements lumineux indésirables; et

un moyen formant photodétecteur (26), couplé optiquement audit moyen formant filtre, pour la détection de l'intensité de ladite émission lumineuse dans ladite bande de longueur d'onde de 1,27 micromètre.

3. Appareil selon la revendication 2, dans lequel ledit moyen formant photodétecteur (26) est une diode photoélectrique à InGaAs.

4. Appareil selon la revendication 2, dans lequel ledit moyen de détection optique (10) comprend en outre un moyen (22, 36, 44) pour recueillir lesdites émissions lumineuses produites dans lesdits tissus pendant la photochimiothérapie, la sortie dudit moyen de recueil étant couplée optiquement audit moyen formant filtre (24).

5. Appareil selon la revendication 4, dans lequel ledit moyen de recueil est une liaison (36) à fibres optiques.

7. Appareil selon la revendication 4, dans lequel ledit moyen de recueil est un agencement de lentille.

8. Appareil selon la revendication 4, dans lequel ledit rayonnement thérapeutique (16) est impulsionnel et dans lequel ledit moyen d'amplification comprend:

un préamplificateur (28), relié audit moyen formant photodétecteur (26), pour amplifier lesdits signaux électriques produits par ledit moyen formant photodétecteur; et

un intégrateur (30) de longues impulsions séparées par de très courts intervalles, relié audit préamplificateur (28) pour transformer la sortie dudit préamplificateur.

9. Appareil selon la revendication 4, dans lequel ledit rayonnement thérapeutique (18) est sensiblement continu, ledit moyen de détection comprend en outre un pulsateur (46) couplé optiquement à la sortie dudit moyen de recueil et couplé optiquement à la sortie dudit moyen formant filtre, et dans lequel ledit moyen d'amplification comprend un amplificateur de blocage (34).

10. Appareil selon la revendication 9, dans lequel ledit pulsateur est un modulateur optoacoustique.

11. Appareil selon la revendication 2, dans lequel ledit rayonnement thérapeutique (18) est sensiblement continu, et ledit moyen de détection optique comprend un pulsateur (46) couplé optiquement à l'entrée dudit moyen formant filtre (24).

12. Appareil selon la revendication 5, dans lequel ladite liaison (36) à fibres optiques est introduite dans lesdits tissus cancéreux via une aiguille creuse hypodermique (40).

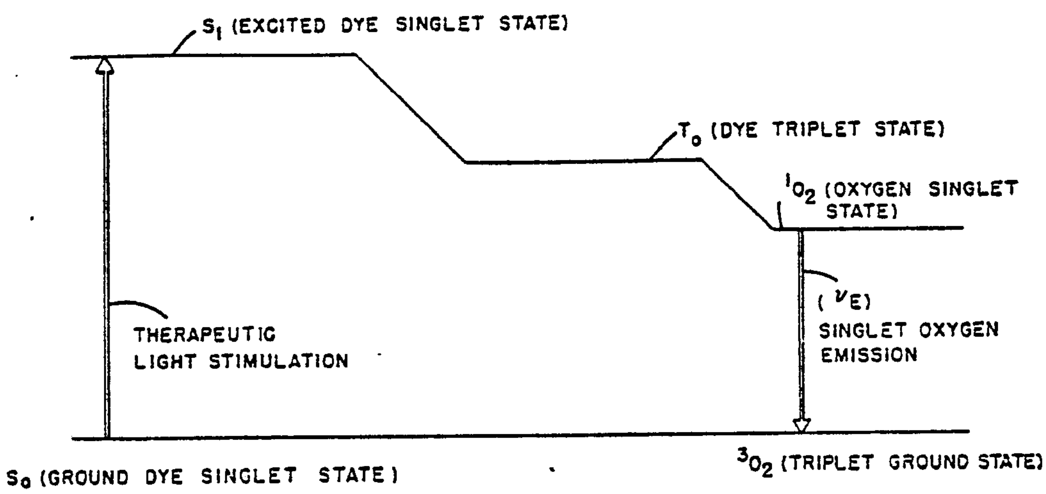


FIG. 1

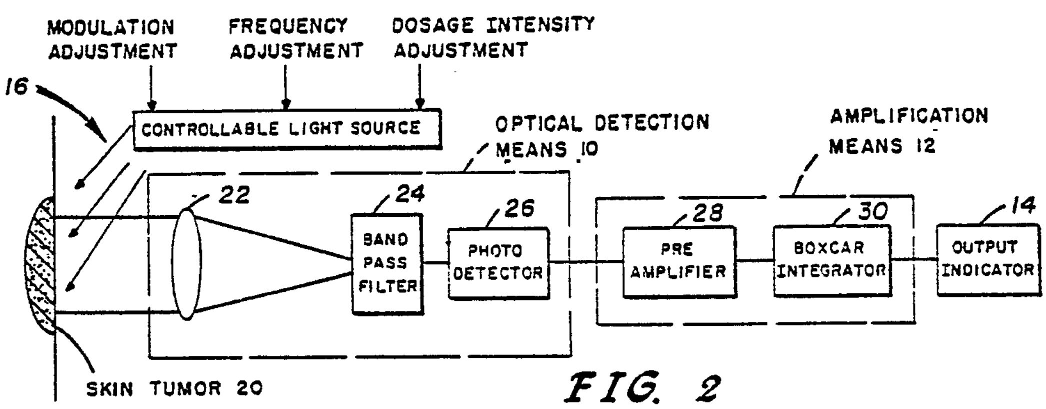


FIG. 2

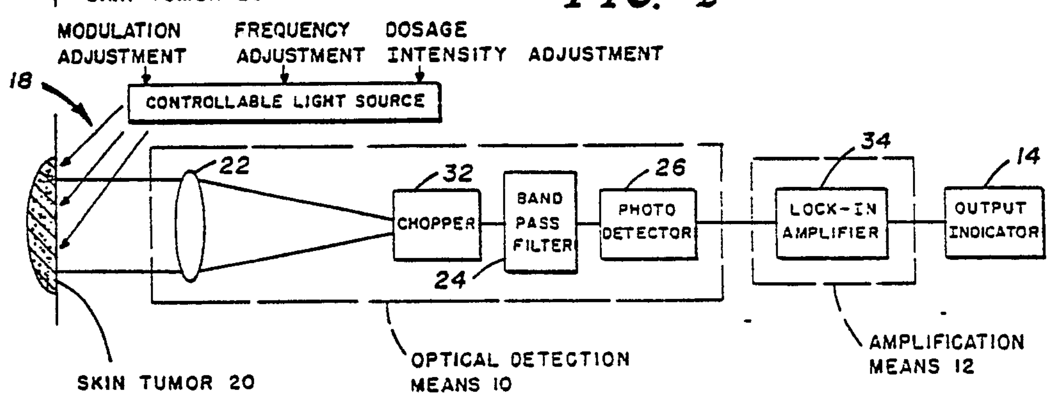


FIG. 3

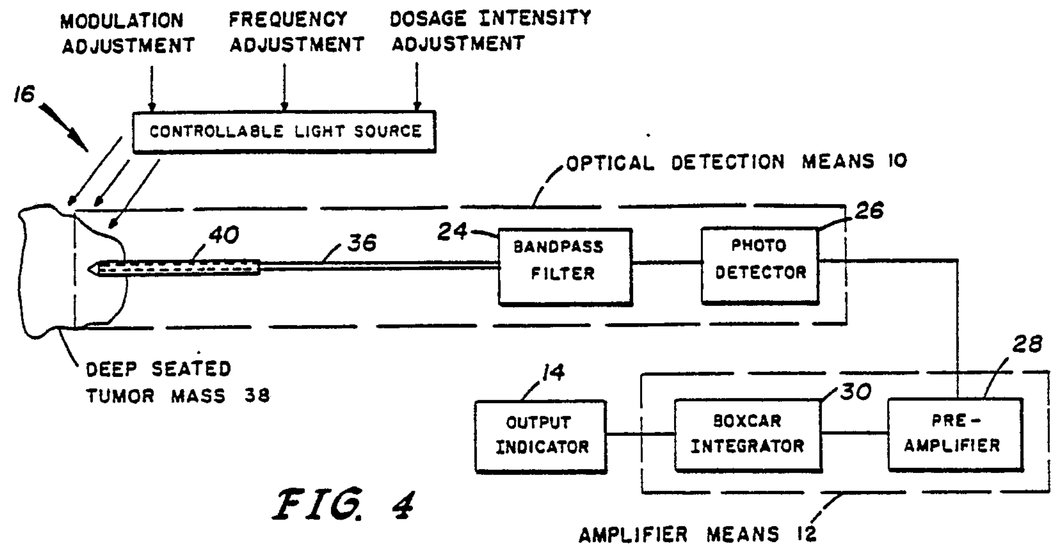


FIG. 4

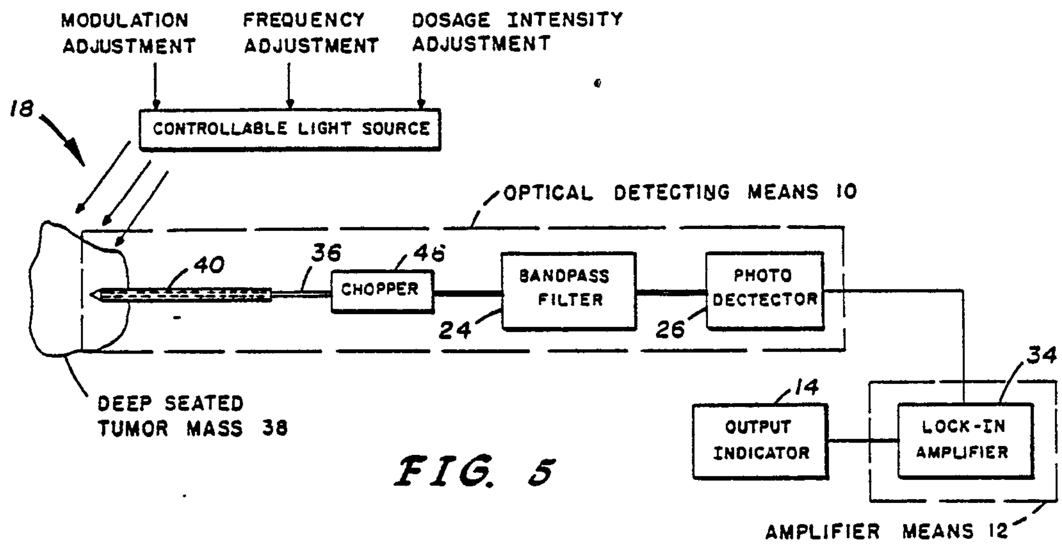


FIG. 5

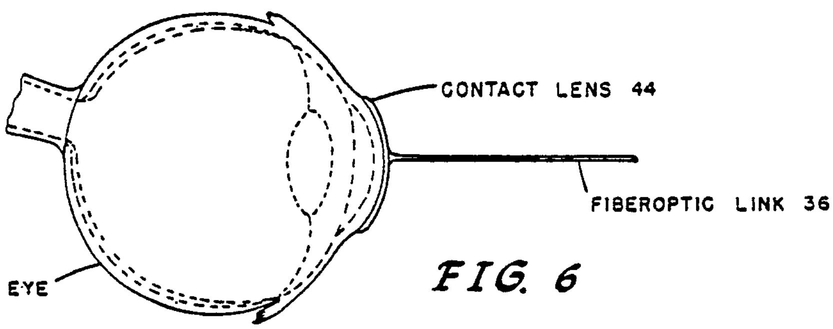


FIG. 6